# EUROPEAN PATENT APPLICATION

(11) **EP 4 578 959 A1**
(43) Date of publication of application: **02.07.2025**
(21) Application number: 23220471.9
(22) Date of filing: 28.12.2023
(51) Int. Cl.: C12Q 1/6816, G01N 33/53, C12N 15/09

(54) **IMMUNOASSAY FOR DETECTION OF SPECIFIC NUCLEIC ACIDS, SUCH AS MRNA OR OTHER LARGE RNA MOLECULES**

(71) Applicant: Siemens Healthineers AG, 91301 Forchheim (DE)
(72) Inventor: Huang, Yiwei, 91058 Erlangen (DE)
(74) Representative: Siemens Healthineers Patent Attorneys

(57) **Abstract**

The present invention relates to a method for detecting a target RNA polynucleotide by using a pool of targeted DNA catcher polynucleotides which generate stretches of RNA/DNA heterohybrids that can be detected with antibodies specific to said RNA/DNA heterohybrids. Further envisaged is a kit for detecting a specific target RNA polynucleotide comprising inter alia one or more DNA catcher polynucleotides complementary to at least a portion of the target RNA polynucleotide, a mature crRNA molecule which is specific for a target sequence on the target RNA polynucleotide and a catalytically-inactive crRNA-guided RNA-binding protein; as well as the use of these ingredients for the detection of the target RNA polynucleotide.

## Description

### TECHNICAL FIELD

The present invention relates to a method for detecting a target RNA polynucleotide by using a pool of targeted DNA catcher polynucleotides which generate stretches of RNA/DNA heterohybrids that can be detected with antibodies specific to said RNA/DNA heterohybrids. Further envisaged is a kit for detecting a specific target RNA polynucleotide comprising inter alia one or more DNA catcher polynucleotides complementary to at least a portion of the target RNA polynucleotide, a mature crRNA molecule which is specific for a target sequence on the target RNA polynucleotide and a catalytically-inactive crRNA-guided RNA-binding protein; as well as the use of these ingredients for the detection of the target RNA polynucleotide.

### BACKGROUND

The field of molecular diagnostics has become increasingly important in clinical laboratories. Various molecular diagnostic tests and assay are used in modern clinical applications, e.g. in the context of infectious diseases, oncological examinations, coagulation tests, pharmacogenetics, or the assessment of genetic disorders (Williams et al., Molecular Pathology 2018, 691-707). Molecular diagnostic testing is usually utilized to detect specific DNA or RNA sequences which are directly or indirectly associated with a disease, including single nucleotide polymorphisms (SNPs), deletions and insertions. Current techniques for detection or quantification of specific nucleic acid sequences include heterogeneous hybridization assays, PCR methods or direct sequencing.

The CRISPR/Cas-technology is a new and very versatile genome- and epigenome-editing tool based on repurposing the CRISPR/Cas (clustered regularly interspersed short palindromic repeats/Cas) bacterial immune system (Cong et al, 2013, Science, 339, 819-824) . The Cas nuclease, when complexed with a short RNA oligonucleotide known as a single guide RNA (sgRNA), can induce double-stranded breaks (DSBs) at specific sgRNA complementary locations.

Recently, the CRISPR/Cas-technology was adapted to provide low-cost and practical diagnostic tools also for RNA targeting. In particular, type VI CRISPR-Cas systems such as the Cas13-based system are capable of binding to RNA polynucleotides when guided by suitable cRNA forms (Murugan et al., 2017, Mol Cell., 68(1), 15-25). An example of a Cas13-based approach is SHERLOCK (Specific High-Sensitivity Enzymatic Reporter UnLOCKing), a diagnostic tool capable of fast and sensitive detection of RNA in samples based on collateral cleavage of reporter release signals (Gootenberg et al., 2017, Science, 360, 6387, 439-444). Koonin et al., 2023, Biochemistry (doi.org/10.1021/acs.biochem.3c00159) describes numerous variants of Cas12 and Cas13 as substrates for the development of versatile, orthogonal molecular tools.

However, the detection techniques typically take a quite long time to result, making them difficult to apply for critical medical situations. They are also complicated, expensive, and low-throughput. Furthermore the tests are normally not suitable to immunoassay analyzers. CRISPR/Cas based approaches, on the other hand, are confined to rather specific situations and do not exploit all diagnostic options provided by the CRISPR/Cas-system. Thus, there is a need for new and improved detection approaches, which allow for an efficient and accurate characterization of RNA polynucleotides.

### SUMMARY

The present invention addresses this need and provides a method for detecting a target RNA polynucleotide, comprising the following steps: (i) providing an RNA polynucleotide; (ii) annealing a mature crRNA (CRISPR RNA) molecule to a specific target sequence on the target RNA polynucleotide; (iii) binding a catalytically-inactive crRNA-guided RNA-binding protein to the complex of step (ii) to obtain a protein-RNA polynucleotide complex, wherein said crRNA-guided RNA-binding protein is biotinylated; (iv) binding a magnetic bead, which comprises one or more streptavidin molecules, to the protein-RNA polynucleotide-complex of step (iii); (v) removing unbound mature crRNA molecule and unbound catalytically inactive crRNA-guided RNA-binding protein; (vi) annealing a pool of targeted DNA catcher polynucleotides to the complex of step (iv), thereby generating stretches of RNA/DNA heterohybrids between the targeted DNA catcher polynucleotides and the target RNA polynucleotide; (vii) specific binding of an antibody to said stretches of RNA/DNA heterohybrids of the complex of step (vi) ; (viii) removing unbound antibody; and (ix) detecting the antibody-bound RNA/DNA heterohybrids of step (vii).

The method is suitable for the detection and/or quantification of RNAs and allows to obtain quantifiable results with an improved dynamic range. In addition, the method is advantageously adaptable to RNA molecules with different sequences and can be easily repurposed to automated laboratory platforms or point-of-care type settings. Further benefits include relatively short procedure times, the adaptability to high-throughput immunoassay analyzers and a high specificity.

In a preferred embodiment of the present invention, said pool of targeted DNA catcher polynucleotides comprises a multitude of DNA fragments which are capable of binding to different portions of the target RNA polynucleotide.

In a particularly preferred embodiment, the pool of targeted DNA catcher polynucleotides comprises different groups of targeted DNA catcher polynucleotides, wherein each group comprises identical targeted DNA catcher polynucleotides and wherein said targeted DNA catcher polynucleotides of said different groups bind to different portions of the target RNA polynucleotide.

In a further embodiment, the different groups of targeted DNA catcher polynucleotide cover 20% to 100% the entire length of the target RNA polynucleotide.

In yet another embodiment, the targeted DNA catcher polynucleotide is at least partially complementary to a specific target sequence on the target RNA polynucleotide.

In a further embodiment of the present invention, the complementary section between the targeted DNA catcher polynucleotide and the target RNA polynucleotide comprises between 12 to 30 nucleotides, preferably 20 nucleotides.

In another embodiment of the present invention, the antibody is specific for RNA/DNA heterohybrids. In a preferred embodiment said antibody is a monoclonal antibody.

In another embodiment of the present invention, the catalytically-inactive crRNA-guided RNA-binding protein is a Cas protein.

In a further embodiment, the Cas protein is a member of the family of Cas12 or Cas13 proteins. It is particularly preferred that the Cas protein is a Cas13 protein or a derivative thereof.

In yet another preferred embodiment of the present invention, the detection of the antibody-bound RNA/DNA heterohybrids of step (vii) is based on an enzymatic or a non-enzymatic reaction.

In a specifically preferred embodiment, the enzymatic reaction is an enzymatic reaction of horseradish peroxidase (HRP), alkaline phosphatase (AP), esterase or glucose oxidase (GOD), wherein said HRP, AP, esterase or GOD is covalently linked to the antibody.

In a further embodiment, the non-enzymatic reaction is a chemiluminescent reaction of an acridinium ester, wherein said acridinium ester is covalently linked to the antibody.

In yet another preferred embodiment of the method of the present invention, said detection is a quantitative detection or a semi-quantitative detection.

In another aspect the present invention relates to a kit for detecting a target RNA polynucleotide comprising one or more DNA catcher polynucleotides which are complementary to at least a portion of the target RNA polynucleotide, a mature crRNA (CRISPR RNA) molecule which is specific for a target sequence on the target RNA polynucleotide, a catalytically-inactive crRNA-guided RNA-binding protein which is guided by said mature crRNA, and an antibody binding to RNA/DNA heterohybrids between said target RNA polynucleotide and said DNA catcher polynucleotides.

In yet another aspect, the present invention relates to the use of one or more DNA catcher polynucleotides which are complementary to at least a portion of a target RNA polynucleotide, a mature crRNA (CRISPR RNA) molecule which is specific for a target sequence on the target RNA polynucleotide, a catalytically-inactive crRNA-guided RNA-binding protein which is guided by said mature crRNA and an antibody binding to RNA/DNA heterohybrids between said target RNA polynucleotide and said DNA catcher polynucleotide(s) for the detection of a target RNA polynucleotide.

It is to be understood that the features mentioned above and those yet to be explained below may be used not only in the respective combinations indicated, but also in other combinations or in isolation without departing from the scope of the invention.

### BRIEF DESCRIPTION OF THE DRAWINGS

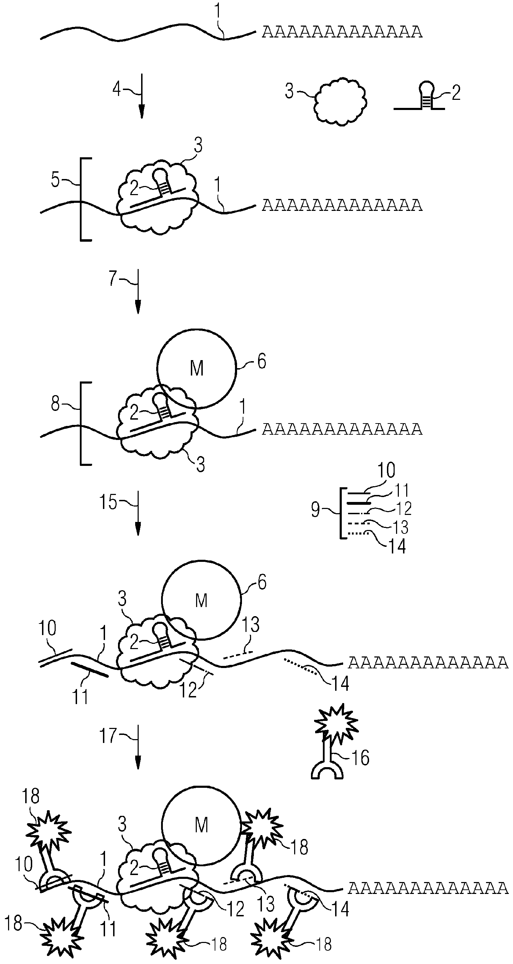

FIG. 1 shows a schematic illustration of the steps for detecting a target RNA polynucleotide according to an embodiment of the present invention.

### DETAILED DESCRIPTION OF EMBODIMENTS

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of" or "essentially consisting of" is considered to be a preferred embodiment of the term "comprising of". If hereinafter a group is defined to comprise at least a certain number of embodiments, this is meant to also encompass a group which preferably consists of these embodiments only.

Furthermore, the terms "(i)", "(ii)", "(iii)" or " (a) ", "(b)", " (c) ", " (d) ", or "first", "second", "third" etc. and the like in the description or in the claims, are used for distinguishing between similar elements and not necessarily for describing a sequential or chronological order.

It is to be understood that the terms so used are interchangeable under appropriate circumstances and that the embodiments of the invention described herein are capable of operation in other sequences than described or illustrated herein. In case the terms relate to steps of a method, procedure or use there is no time or time interval coherence between the steps, i.e. the steps may be carried out simultaneously or there may be time intervals of seconds, minutes, hours, days, weeks etc. between such steps, unless otherwise indicated.

It is to be understood that this invention is not limited to the particular methodology, protocols etc. described herein as these may vary. It is also to be understood that the terminology used herein is for the purpose of describing particular embodiments only, and is not intended to limit the scope of the present invention that will be limited only by the appended claims.

The drawings are to be regarded as being schematic representations and elements illustrated in the drawings are not necessarily shown to scale. Rather, the various elements are represented such that their function and general purpose become apparent to a person skilled in the art.

Unless defined otherwise, all technical and scientific terms used herein have the same meanings as commonly understood by one of ordinary skill in the art.

Independent of the grammatical term usage, individuals with male, female or other gender identities are included within the term.

As has been set out above, the present invention concerns in one aspect a method for detecting a target RNA polynucleotide, comprising the following steps: (i) providing an RNA polynucleotide; (ii) annealing a mature crRNA (CRISPR RNA) molecule to a specific target sequence on the target RNA polynucleotide; (iii) binding a catalytically-inactive crRNA-guided RNA-binding protein to the complex of step (ii) to obtain a protein-RNA polynucleotide complex, wherein said crRNA-guided RNA-binding protein is biotinylated; (iv) binding a magnetic bead, which comprises one or more streptavidin molecules, to the protein-RNA polynucleotide-complex of step (iii); (v) removing unbound mature crRNA molecule and unbound catalytically inactive crRNA-guided RNA-binding protein; (vi) annealing a pool of targeted DNA catcher polynucleotides to the complex of step (iv), thereby generating stretches of RNA/DNA heterohybrids between the targeted DNA catcher polynucleotides and the target RNA polynucleotide; (vii) specific binding of an antibody to said stretches of RNA/DNA heterohybrids of the complex of step (vi); (viii) removing unbound antibody; and (ix) detecting the antibody-bound RNA/DNA heterohybrids of step (vii) .

In a first step of the method of the present invention thus an RNA polynucleotide is provided.

As used herein the term "RNA polynucleotide" or "target RNA polynucleotide" relates to any macromolecule comprising two or more ribonucleotides. Ribonucleotides typically contain a nucleobase, a ribose sugar and at least one phosphate group. The nucleobases are typically adenine, guanine, cytosine and uracil. RNA polynucleotides are typically single-stranded molecules, can, however, also be provided in a double-stranded form by partial complementary base pairing. The RNA polynucleotide typically does not form long double helical stretches. The RNA polynucleotide may be naturally occurring or be artificial. It may comprise, in addition to the elements mentioned above, modifications such as oxidized or methylated nucleotides. The RNA polynucleotide may also, in certain embodiments, comprise artificial additions such as tags or labels.

The RNA polynucleotide may be of any possible origin, e.g. prokaryotic, eukaryotic, archaeal or viral. The RNA polynucleotide to be characterized according to the present invention may have any known possible biological or cellular function. For example it may be any naturally occurring or synthetic polynucleotide such as messenger RNA (mRNA), ribosomal RNA (rRNA), heterogenous nuclear RNA (hnRNA), transfer RNA (tRNA), transfer messenger RNA (tmRNA), precursor microRNAs (pre-miRNA), small nuclear RNA (snRNA), spliced leader RNA (SL RNA), small nucleolar RNA (snoRNA), antisense RNA (asRNA), guide RNA (gRNA), long noncoding RNA (lncRNA), small interfering RNA (siRNA), Piwi-interacting RNA (piRNA), trans-acting RNA (tasiRNA), precursor mRNA (pre-mRNA) or repeat associated siRNA (rasiRNA), especially viral RNAs. The RNA species mentioned typically differ in terms of size, nucleotide composition, folding status and presence or absence of 3' extensions. For example, mRNA molecules in eukaryotes typically comprise a 3' poly-A tail which consists of multiple adenosine monophosphates and is attached to the molecules after transcription by a polyadenylate polymerase. Other RNA species or fragments of eukaryotic mRNAs may not comprise such an elongation. For example, precursor mRNAs (pre-mRNAs) are non-processed mRNA molecules comprising intron sequences which have not yet been modified by the polyadenylate polymerase. In a preferred embodiment, the target RNA polynucleotide is an mRNA, a fragment of an mRNA, pre-mRNA molecule, an ncRNA, a pre-miRNA, an snoRNA, a tmRNA, an siRNA or a piRNA, especially viral RNA It is preferred that the target RNA is an RNA polynucleotide which comprises a poly-A tail or an oligo-A tail at the 3' end. Also envisaged are fragments of mRNA polynucleotides which do not comprise the mRNA 3' portion or do not comprise a poly-A tail. Further envisaged are pre-mRNA molecules which typically do not comprise a poly-A tail. Finally, also envisaged are RNA polynucleotide species that do not belong to the group of eukaryotic mRNAs such as, for exmaple, viral RNA molecules; these species typically do also not comprise poly-A extensions.

The provision of the RNA polynucleotide may include the extraction and/or purification of the RNA molecule, e.g. by guanidine-isothiocyanate lysis, separation from cell debris, filtration, elution from a column, e.g. silica membrane columns, centrifugation, digestion, e.g. DNase digestion, or removal or nucleotide or protein components in a sample etc. It is preferred that the RNA polynucleotide is provided in a buffer solution comprising any suitable ingredient preventing RNA degradation. The buffer may, for example, be an RNAse-free H₂O buffer comprising EDTA (e.g. 0.1 mM) or a TE buffer (10mM Tris, 1mM EDTA) . The buffer may preferably comprise RNAse blocking compounds or RNase inhibitors such as RNaseZap, Superase, RNaseOUT, ribonuclease inhibitor, RNasin or the like.

The RNA polynucleotide may typically be present in a mixture of other molecules, e.g. of other RNA polynucleotides or other nucleic acids. The other RNA polynucleotides or nucleic acids may have any proportion or be present in any amount, typically in any amount or proportion found in natural situations, e.g. found in cells or samples, or found after typical enrichment steps as mentioned herein.

In further embodiments, the provision of RNA polynucleotides may also include the employment of suitable conditions to keep the RNA molecule or a part of it in a single stranded form. Such conditions may include the use of a certain temperature or range of temperatures before or during the performance of the methods of the invention, and/or the use of specific buffers or salt conditions in order to avoid the formation of secondary structures in the RNA molecule. It is also envisaged that the RNA polynucleotide or a part of it is made single stranded by a short heating denaturation. This short heating denaturing may, for example, be applied when annealing a DNA catcher polynucleotide as described herein.

The provision of RNA polynucleotides may also involve the taking of samples from a subject and their processing, e.g. extraction of RNA or preparatory steps facilitating the extraction of RNA. The term "sample from a subject" as used herein relates to any biological material obtained via suitable methods known to the person skilled in the art from a subject. The sample used in the context of the present invention should preferably be collected in a clinically acceptable manner, more preferably in a way that RNA polynucleotides are preserved. The biological samples may include body tissues and/or fluids, such as blood, or blood components like serum or plasma, sweat, sputum or saliva, semen and urine, as well as feces or stool samples. Furthermore, the biological sample may contain a cell extract derived from or a cell population including an epithelial cell, preferably a neoplastic epithelial cell or an epithelial cell derived from tissue suspected to be neoplastic, or a cancerous cell or cancerous tissue. Alternatively, the biological sample may be derived from the environment, e.g. from the soil, a lake, a river etc., or from animal sources.

In certain embodiments cells or viruses may be used as primary sources for RNA polynucleotides. Accordingly, the cells may be purified from obtained body tissues and fluids if necessary, and then further processed to obtain RNA polynucleotides. In certain embodiments samples, in particular after initial processing, may be pooled. The present invention preferably envisages the use of non-pooled samples.

In a specific embodiment of the present invention the content of a biological sample may also be submitted to an enrichment step. For instance, a sample may be contacted with ligands specific for the cell membrane or organelles of certain cell types, functionalized for example with magnetic particles. The material concentrated by the magnetic particles may subsequently be used for the extraction of RNA polynucleotides. In further embodiments of the invention, biopsy or resections samples may be obtained and/or used. Such samples may comprise cells or cell lysates. Furthermore, cells, e.g. tumor cells, may be enriched via filtration processes of fluid or liquid samples, e.g. blood, urine, sweat etc. Such filtration processes may also be combined with enrichment steps based on ligand specific interactions as described herein above.

The RNA polynucleotides are typically provided in a liquid, e.g. aqueous, solution. The solution may comprise or be composed of suitable buffers such as a annealing/hybridization buffer, e.g. comprising SSC, NaCl, sodium phosphate, SDS, TE and/or MgCl₂.

In a next step a mature crRNA (CRISPR RNA) molecule is annealed to a specific target sequence on the target RNA polynucleotide. The specific target sequence for the crRNA is typically not identical to the specific target sequence for the DNA catcher polynucleotide. It is also preferred that these sequence segments do not overlap.

This method is, in general, based on the employment of the CRISPR/Cas system. The term "CRISPR/Cas system" as used herein relates to a biochemical method to specifically cut and modify nucleic acids, also known as genome editing. For example, genes in a genome can generally be inserted, removed or switched off with the CRISPR/Cas system, nucleotides in a gene or nucleic acid molecule can also be changed. The effect of the concept and activity steps of the CRISPR/Cas system has various similarities to that of RNA interference, since short RNA fragments of about 18 to 20 nucleotides mediate the binding to the target in both bacterial defense mechanisms. In the CRSIPR/Cas system typically RNA-guided nucleic acid-binding proteins, such as Cas proteins, bind certain RNA sequences as ribonucleoproteins. For example, a Cas endonuclease can bind to certain RNA sequences termed crRNA repeats and cut a nucleic acid in the immediate vicinity of these sequences. Without wishing to be bound by theory, it is believed that the crRNA repeat sequence forms a secondary RNA structure and is then bound by the nucleic acid-binding protein (e.g. Cas) which alters its protein folding allowing the target nucleic acid to be bound by the RNA.

In type VI CRISPR/Cas systems a single RNA-guided effector protein, Cas13, forms a crRNA-guided RNA-targeting effector complex when assembled with a crRNA. The CRISPR/Cas13 system accordingly comprises two main components: (i) the programmable single-effector RNA-guided RNase Cas13 and (ii) a 64-66 nucleotide CRISPR RNA (crRNA), which recognizes a 24-30 nucleotide sequence on the target RNA by means of a protospacer-flanking site (PFS). Also, all Cas13 proteins feature two enzymatically distinct ribonuclease activities: (i) nucleolytical processing of pre-crRNA pre-CRISPR-RNA by Cas proteins and/or host factors to generate mature crRNA by RNase and (ii) degradation of target RNA initialized by the RNase activity of two HEPN (higher eukaryotes and prokaryotes nucleotide-binding) domains (Grandaros-Riveron and Aquino-Jarquin, 2018, Cancer Research, 78(15), 4107-13).

The term "annealing" as used herein generally relates to the pairing of a single stranded RNA polynucleotide with a complementary single stranded polynucleotide by hydrogen bonds to form a double-stranded polynucleotide. The complementary polynucleotide may either be an RNA molecule, as in the case of the mature crRNA (CRISPR RNA) molecule, or a DNA polynucleotide. It may further comprise one or more modifications such as comprising one or more non-naturally occurring nucleotides, chemical modifications to the nucleotides, the presence of tag or spacer elements etc. Nucleotides or nucleotide analogues will bind to their complement under normal conditions, so two complementary strands will bind to each other readily. Annealing can be e.g. performed in a PCR machine or similar heating device which allows to select a predetermined temperature for annealing. If such a heating device allows the application of a temperature gradient across the field of the microtiter plate, an optimal annealing temperature can be quickly determined.

Annealing typically takes place in a liquid solution, e.g. an aqueous solution comprising a suitable buffer as defined above. The annealing may be performed in accordance with any suitable temperature, ion concentration and/or pH parameter known to the skilled person. For example, the annealing may be performed at a temperature and/or pH and/or ionic concentration in the solution at which a complementary base-pairing between most, preferably all complementary bases in the target RNA polynucleotide and the DNA catcher polynucleotide takes place. Unspecific binding or annealing reactions may, for example, be avoided by setting the temperature to a value which only allows for a complete, i.e. 100% complementary binding. Alternatively, the temperature may be set to a value, which allows for a complementary binding of about 99%, 98%, 95%, 90%, 85% or 80% of complementary bases.

The term "mature crRNA (CRSIPR RNA) " as used herein thus relates to an RNA molecule of the type VI CRISPR/Cas system which is capable of identifying and binding to specific RNA polynucleotide sequences. Typically, the mature crRNA comprises a target specific sequence which can be used to guide an RNA binding protein towards the binding site. This target specific sequence may have any suitable length. It is preferred that said length is between about 19 to 60 nucleotides. More preferably, the sequence has a length of 30 nucleotides.

As described in East-Seletsky et al., 2017, Mol Cell., 66(3), 373-383 mature crRNAs of the type VI CRISPR/Cas system according to the present invention comprise a stem segment, a loop segment, a bulge segment and a spacer segment which comprises the target specific sequence as defined above. Mature crRNAs typically are derived from pre-crRNA molecules via a maturation processing. They can also be provided as such and be used without being derived from said pre-crRNA stage. Further options for obtaining a mature crRNA can be derived from Smargon et al., 2017, Mol Cell., 16, 65(4), 618-630, from Cox et al., 2017, Science, 10.1126/science.aaq0180, or from Smargon et al., 2020, Nat. Cell. Biol., 22 (2), 143-150. For example, a mature crRNA may be provided which comprises different forms of a stem structure, different loop structures, or in which the spacer sequence is differentially truncated etc. According to further embodiments of the present invention, the sequence and form of the mature crRNA may vary in accordance with the form or identity of the crRNA-guided RNA-binding protein, e.g. Cas protein. Accordingly, depending on the origin of said crRNA-guided RNA-binding protein, a different combination of sequence elements may be used. The present invention further envisages any future development in this context and includes any modification or improvement of the crRNA-RNA-binding protein interaction surpassing the information derivable from Smargon et al., 2017, Mol Cell., 16, 65(4), 618-630 or Mendez-Mancilla et al., 2022, Cell Chemical Biology, 29, 321-327. Particularly preferred is the use of a mature crRNA for Cas13a, Cas13b, Cas13c or Cas13d, or modifications thereof. Also envisaged are mature crRNA forms from commercial suppliers, or individually prepared crRNAs.

One of the central principles of the present invention is thus the use of a sequence binding to a target RNA section within the crRNA, wherein said binding sequence is specific for the target RNA polynucleotide and is accordingly able to identify said sequence and to distinguish it from other sequences.

According to further specific embodiments of the invention said annealing of a mature crRNA (CRISPR RNA) molecule to a specific target sequence on the target RNA polynucleotide may take place at more than one specific site, e.g. at 2, 3, 4, 5, 6 or more sites. In accordance with this embodiment a corresponding number of different mature crRNA (CRISPR RNA) molecules may be used in the step (ii), each being specific for a different target sequence on the target RNA polynucleotide. The different target sequences may be located anywhere along the length of the target RNA polynucleotide, e.g. at the 5' end, at the 3' end or in a region between the 5' end and the 3' end etc. In a preferred example, the 2, 3, 4, 5, 6 or more specific sites may be located within a virus RNA. By employing more than one different mature crRNA (CRISPR RNA) molecule the method according to the present invention allows for a detection of target RNA polynucleotides also in cases in which the target RNA polynucleotide has been partially degraded or destroyed.

It is preferred that the target sequence in the target RNA polynucleotide is a sequence which is evolutionary conserved or which is known to show a minimum mutational frequency, e.g. reflecting conversed or essential protein structures or functions etc. in order to minimize the probability of or avoid mismatches in the binding process between the mature crRNA (CRISPR RNA) molecule and the target RNA polynucleotide. In a further step a catalytically-inactive crRNA-guided RNA-binding protein is bound to the complex of step (ii), i.e. the complex of a mature crRNA (CRISPR RNA) molecule and the target RNA polynucleotide. This results in a protein-RNA polynucleotide complex. In accordance with the CRSPR/Cas approach as defined above, the sequence which has been identified by the mature crRNA as described above is thus indirectly bound (via the crRNA) by a cognate catalytically-inactive crRNA-guided RNA-binding protein. Said crRNA-guided RNA-binding protein is further equipped with an interactor component, preferably it is biotinylated and is thus capable of interacting with a cognate binding partner which comprises a streptavidin component or a functional equivalent thereof.

The term "catalytically-inactive crRNA-guided RNA-binding protein" as used herein relates to an RNA binding protein, e.g. Cas protein, which is capable of binding to a specific stretch of RNA, but does not retain its endonuclease activity and thus does not cut or digest the RNA. This allows for a programmable tracking and identification of RNA molecules without damaging them. In a preferred embodiment, said catalytically-inactive crRNA-guided RNA-binding protein is a Cas protein, more preferably it is a member of the family of Cas12 or Cas13 proteins. Examples of proteins of the Cas12 family include Cas12a. The Cas12 protein is preferably a derivative of the naturally occurring Cas12 protein, in particular a derivative which has been modified, e.g. via protein engineering, to be capable of binding to RNA polynucleotides. It is particularly preferred that it is a Cas13 protein or a derivative thereof. Suitable subtypes may include, for example, Cas13a, Cas13b, Cas13c and Cas13d proteins. Further information on catalytically-inactive crRNA-guided RNA-binding protein may be derived from suitable literature sources such as Cox et al., 2017, Science, 10.1126/science.aaq0180.

Also envisaged are derivatives of the mentioned RNA-binding proteins, e.g. Cas proteins, or mutants thereof. The derivative is preferably a functional derivative of a crRNA-guided RNA-binding protein, which is able to bind to specific stretches of RNA but does no retain its endonuclease activity. In specific embodiments the invention particularly envisages the production of catalytically inactive Cas13 or Cas12 proteins or "dead" Cas proteins (dCas) . These proteins can be generated according to any suitable method known to the skilled person. For example, they may be obtained by mutating catalytic arginine residues within the two conserved HEPN domains. Further information may be derived from Abudayyeh et al., 2017, Nature, 550(7675), 280-284 or Cox et al., 2017, Science, 10.1126/science.aaq0180.

The present invention also envisages the use of Cas proteins, e.g. Cas13, derived from different bacterial sources. For example, the Cas13 protein may be derived from Leptotrichia shahii, Leptotrichia wadei, Prevotella spp., or Streptococcus pyogenes.

Further details on the form and use of Cas proteins may be derived from suitable literature sources such as Granados-Riveron and Aquino-Jarquin, 2018, Cancer Research; Cox et al., 2017, Science, 10.1126/science.aaq0180; Gootenberg et al., 2018, Science 360, 439-444 or Abudayyeh et al., 2016, Science, 353(6299).

In a subsequent step a magnetic bead is bound to the protein-RNA polynucleotide-complex of step (iii). The present invention accordingly envisages that the magnetic bead comprises a tag which is capable of binding to a cognate interactor present in the catalytically-inactive crRNA-guided RNA-binding protein. The interactor-tag binding may advantageously be used to pull said catalytically-inactive crRNA-guided RNA-binding protein together with any bound or associated RNA polynucleotide from a solution, or to catch a target RNA polynucleotide when moving around said DNA catcher polynucleotide. Examples of suitable tags and interactors are a biotin tag on the catalytically-inactive crRNA-guided RNA-binding protein and a streptavidin interactor provided on the magnetic bead. The present invention further envisages the presence of a magnetic separator attracting said beads, e.g. attached at a surface. In alternative embodiments, the streptavidin interactor may also be provided on suitable surfaces, e.g. of a reaction vessel.

In preferred embodiments, the magnetic bead comprises one or more streptavidin molecules, which can interact with one or more biotin molecules present at the surface of the catalytically-inactive crRNA-guided RNA-binding protein. In particularly preferred embodiments, the amount of streptavidin is 250 to 5000 pmole streptavidin per mg magnetic bead.

The binding of the magnetic bead to the protein-RNA polynucleotide-complex of step (iii) may preferably be followed, e.g. after an actuation of a magnetic separator attracting said beads to an immobilization of the complex, for instance at a surface.

In a subsequent step unbound mature crRNA molecules and unbound catalytically inactive crRNA-guided RNA-binding proteins are removed. The removal may be implemented with any suitable technique, e.g. by removing or relocating the reactants, inhibiting or destroying crRNA molecules or RNA binding proteins etc. In a typical and preferred embodiment, the removal is achieved by washing the complex as obtained in the previous step(s) of the method. The washing step may be performed once, or it may be repeated 1, 2, 3 or more times. It is preferred to perform the washing with nuclease-free water or with any other suitable solution containing appropriate ion concentration and/or having a suitable pH, as would be known to the skilled person.

In a subsequent step of the method a pool of targeted DNA catcher polynucleotides is annealed to the complex of step (iv), thereby generating stretches of RNA/DNA heterohybrids between the targeted DNA catcher polynucleotides and the target RNA polynucleotide.

The term "targeted DNA catcher polynucleotide" as used herein relates to a polynucleotide of a defined length which is complementary to at least a portion of the target RNA polynucleotide and which is designed to anneal with and thereby to bind to or to catch a corresponding target RNA polynucleotide.

The targeted DNA catcher polynucleotide may have any suitable length. It may, for example, comprise between about 12 to 300 nucleotides, e.g. 12, 13, 14, 15, 16, 17, 18, 19, 20, 21, 22, 23, 24, 25, 26, 27, 28, 29, 30, 40, 50, 100, 150, 200, 250, or 300 nucleotides or any value in between the mentioned values. Also envisaged are longer targeted DNA catcher polynucleotides. It is further preferred that the targeted DNA catcher polynucleotide comprises about 12-30 nucleotides. It is particularly preferred that the targeted DNA catcher polynucleotide comprises about 20 nucleotides. The targeted DNA catcher polynucleotide may, in certain embodiments, further comprise additional elements such as one or more spacer elements, linker elements etc. The targeted DNA catcher polynucleotide may, in further specific embodiments, comprise one or more sections which are capable of binding or hybridizing to a target RNA polynucleotide and one or more segments which are not capable of binding or hybridizing to a target RNA polynucleotide. The latter ones may have structural functions or provide a spacer or distancing functionality. Typically, the targeted DNA catcher polynucleotide is a single strand DNA molecule. In certain specific embodiments, the targeted DNA catcher polynucleotide may comprise one or more non-DNA nucleotides such as PNA, CNA, HNA, LNA or ANA molecules.

The term "complementary", as used herein, refers to the presence of matching base pairs in opposite nucleic acid strands. For example, to a nucleotide or base A in a sense strand a complementary or antisense strand binds with a nucleotide or base T, or vice versa; likewise to a nucleotide or base G in a sense strand the complementary or antisense strand binds with a nucleotide or base C, or vice versa. This scheme of complete or perfect complementarity may, in certain embodiments of the invention, be modified by the possibility of the presence of single or multiple non-complementary bases or stretches of nucleotides within the sense and/or antisense strand(s). Thus, to fall within the notion of a pair of sense and antisense strands, both strands may be completely complementary or may be only partially complementary, e.g. show a complementarity of about 80%, 85%, 90%, 91%, 92%, 93%, 94%, 95%, 96%, 97%, 98%, 99%, 99.5%, or 100% between all nucleotides of both strands or between all nucleotides in specific segments as defined herein. Non-complementary bases may comprise one of the nucleotides A, T, G, C, i.e. show a mismatch e.g. between A and G, or T and C, or may comprise any modified nucleoside bases including, for example, modified bases as described in WIPO Standard ST.26.

The term "complementary to at least a portion of the target RNA polynucleotide" as used herein, means that the annealing segment has a complementary overlap with said target RNA polynucleotide. For example, the targeted DNA catcher polynucleotide may be complementary to a target RNA polynucleotide or a polynucleotide comprising a target specific sequence as defined herein. The overlap may, for example, be an overlap of 5, 6, 7, 8, 9, 10, 11, 12, 13, 14, 15, 16, 17, 18, 19, 20, 22, 25, 28 or 30 nucleotides, or any value in between the mentioned values. Also envisaged are longer overlaps. The overlap may be at the 3' end of targeted DNA catcher polynucleotide and at the 3' end of the target RNA polynucleotide, or at the 5' end of the targeted DNA catcher polynucleotide and the 5' end of the target RNA polynucleotide. Within said overlap the matching or complementarity between the complementary bases is preferably 100%. Alternatively, the matching is less than 100%, e.g. 99%, 95%, 90%, 85% or less than 85%. A "portion" of the target RNA polynucleotide which is bound by a targeted DNA catcher may accordingly have any suitable length, e.g. 12 to 60 nucleotides, preferably, 12 to 30 nucleotides, more preferably about 20 nucleotides.

Depending on the length of the targeted DNA catcher polynucleotide the binding of said targeted DNA catcher polynucleotide to the complex of step (iv), in particular to the target RNA polynucleotide, leads to the generation of stretches of RNA/DNA heterohybrids between the targeted DNA catcher polynucleotides and the target RNA polynucleotide. A "stretch" of RNA/DNA heterohybrid may accordingly be a double stranded hybrid of a portion of the target RNA polynucleotide and the targeted DNA catcher polynucleotide or a part of it, e.g. if the targeted DNA catcher polynucleotide is only partially complementary to the target RNA polynucleotide or comprises non-complementary sections. It is preferred that the RNA/DNA heterohybrid comprises a complementarity of about 95% to 100%, e.g.95%, 96%, 97%, 98%, 99%, 99.5%, or 100% between all nucleotides of both strands or between all nucleotides in specific segments of both strands. It is particularly preferred that the RNA/DNA heterohybrid comprises a complementarity of 100%.

The stretches of RNA/DNA heterohybrids between the targeted DNA catcher polynucleotides and the target RNA polynucleotide may be localized throughout the entire length of the target RNA polynucleotide, except for the section where the mature crRNA (CRISPR RNA) molecule binds to target RNA polynucleotide. For example, the stretches may be localized in the 3' region, the 5' region or the middle region of the target RNA polynucleotide. In further embodiments, the stretches may cover any suitable length of the target RNA polynucleotide, e.g. the stretches may cover 20% to 100% the entire length of the target RNA polynucleotide, e.g. 20%, 30%, 40%, 50%, 60%, 70, 80%, 90%, 95%, 100% or any value in between the mentioned values, except for the section where the mature crRNA (CRISPR RNA) molecule binds to target RNA polynucleotide.

The term "pool of targeted DNA catcher polynucleotides" as used herein refers to a multitude of DNA fragments, preferably a multitude of DNA fragments which are complementary to the target RNA polynucleotide. In further preferred embodiment, the pool of targeted DNA catcher polynucleotides comprises a multitude of DNA fragments which are capable of binding to different portions of the target RNA polynucleotide. The term "multitude of DNA fragments" as used herein means that at least 2 or more targeted DNA catcher polynucleotides are present in the pool which bind to different portions of the target RNA polynucleotide. In typical embodiments, the pool of targeted DNA catcher polynucleotides comprises several targeted DNA catcher polynucleotides which have a length of about 12-30 nucleotides and which bind to different portions of the target RNA polynucleotide being complementary thereto, e.g. also having a length of about 12-30 nucleotides. Of these differently binding DNA fragments two or more copies may be present in the pool. It is preferred that at least 1000 copies, preferably 5000 up to 100000 copies of identical DNA fragments are present in the pool.

The portions of the target RNA polynucleotide which are bound by the targeted DNA catcher polynucleotides may be directly adjacent, or comprise gaps of one or more, e.g. 1-100 nucleotides between binding targeted DNA catcher polynucleotides, or they may be overlapping, e.g. by 1-5 nucleotides. The portions of the target RNA polynucleotide which are bound by the targeted DNA catcher polynucleotides may, in further embodiments, be selected according to certain rules, e.g. according to known or suspected motives in the target RNA polynucleotide, known or suspected mutational sites, structural motives, the presence of modifications, regulatory sequences, binding sites, sites for viral RNA detection, sites of importance for oncological or neurological screenings or diagnoses etc. Accordingly, the portion of the stretches of generated stretches of RNA/DNA heterohybrids may be only a sub-portion of the entire length of the target RNA polynucleotide, e.g. only portions of interest, which can be selected when designing the pool of DNA catcher polynucleotides.

In further embodiments of the present invention said pool of targeted DNA catcher polynucleotides comprises different groups of targeted DNA catcher polynucleotides, wherein each group comprises identical targeted DNA catcher polynucleotides and wherein said targeted DNA catcher polynucleotides of said different groups bind to different portions of the target RNA polynucleotide. The groups of targeted DNA catcher polynucleotides may comprise any suitable number of copies of the targeted DNA catcher polynucleotides, e.g. at least 100 copies, preferably 5000 to 50000 copies of targeted DNA catcher polynucleotides, in certain preferred embodiments up to 100000 copies. These copies are typically identical or may comprise divergencies of 1-3 nucleotides, e.g. due to copying mistakes during the production. The polynucleotides within different groups may have the same length or different lengths, e.g. any length of a targeted DNA catcher polynucleotides defined herein. The polynucleotides within different groups may, in addition or alternatively, comprises the same or a different degree of complementarity with the target RNA polynucleotide, e.g. any degree of complementarity with the target RNA polynucleotide mentioned herein. The different groups may, in further embodiments, comprise the same or almost the same number or different numbers of polynucleotides.

Said different groups of targeted DNA catcher polynucleotide may cover any portion of the entire length of the target RNA polynucleotide, preferably 20% to 100% the entire length of the target RNA polynucleotide, more preferably 80 to 100%, except for the section where the mature crRNA (CRISPR RNA) molecule binds to target RNA polynucleotide.

The pool of targeted DNA catcher polynucleotide may be produced according to any suitable method. For example, the following workflow may be used: as first step a target RNA polynucleotide is reverse transcribed into a target cDNA pool. The step may be performed with the use of biotinyl-reverse transcription primers. Subsequently, the cDNA pool may be amplified into PCR products, preferably using biotinyl reverse transcription primers and other targeted forward primers without biotin label. The obtained PCR products may then be treated with streptavidin-coated beads and heated. This results in biotinyl-single stranded cDNA amplificates. In last step, the cDNA molecules are fragmented, e.g. into fragments of 12 to 30 nucleotides, preferably into fragments of 20 to 30 nucleotides. By using specific primers the binding region, the binding coverage and/or localization of the DNA catcher polynucleotides on the target RNA polynucleotide may be adjusted. The fragmentation process leads to the provision of groups of DNA catcher polynucleotides which have different binding regions within the target RNA polynucleotide. The obtained DNA catcher polynucleotides may be used directly or stored according to any suitable method.

In a further step of the method according to the present invention, an antibody binds to the stretches of RNA/DNA heterohybrids of the complex of step (vi).

The term "antibody" as used herein refers to immunoglobulin molecules and immunologically active portions of immunoglobulin molecules, i.e. molecules that contain an antigen binding site that immunospecifically binds an antigen, in particular specifically binds the protein-RNA polynucleotide complex as defined herein above, more specifically binds the catalytically inactive crRNA-guided RNA-binding protein being part of said complex. The immunoglobulin molecules of the invention can be of any type (e.g., IgG, IgE, IgM, IgD, IgA and IgY), class (e. g., IgG1, IgG2, IgG3, lgG4, lgA1 and IgA2) or subclass of immunoglobulin molecule. The immunospecific binding refers to the immunospecific detection and binding of an antibody to an antigenic epitope of a catalytically inactive crRNA-guided RNA-binding protein as defined herein above. In further embodiments antibodies of the invention include polyclonal, monoclonal, multispecific, human, humanized or chimeric antibodies, or whole immunoglobulin molecules.

It is preferred that the antibody is a monoclonal antibody. In a particularly preferred embodiment, the antibody according to the present invention is a monoclonal antibody capable of binding an RNA/DNA heterohybrid. According to certain embodiments of the present invention the binding of the RNA/DNA heterohybrid is a non-sequence specific binding, i.e. it does not depend on the sequence of the DNA catcher polynucleotide and/or the target RNA polynucleotide and there is no bias towards a specific sequence. The binding is typically specific for the structure, which is - sequence independently - provided by an RNA/DNA heterohybrid.

According to certain embodiments, the antibody may bind only to perfectly matched RNA/DNA heterohybrids and may not bind to mismatched heterohybrids.

The antibody may, in certain embodiments, also be or comprise an antibody substructure. The term "antibody substructure" as used herein refers to single chain antibodies, Fab fragments, Fab' fragments, fragments produced by a Fab expression library, F(ab')2, Fv, disulfide linked Fv, minibodies, diabodies, scFv, sc(Fv)2, and epitope-binding fragments of any of the above. Preferred are Fab, Fab' and F (ab')2, Fv, single-chain Fvs (scFv), sc(Fv)2, single-chain antibodies, disulfide-linked Fvs (sdFv) and fragments comprising either a VL or VH domain. The term "Fab fragment" as used herein refers to antibody fragments consisting of the first constant domain of the heavy chain (CH1), the constant domain of the light chain (CL), the variable domain of the heavy chain (VH) and the variable domain of the light chain (VL) of an intact immunoglobulin protein.

The term "F(ab')2" or "F(ab')2 fragment" as used herein refers to antibody fragments consisting of two first constant domains of the heavy chain (CH1), two constant domains of the light chain (CL), two variable domains of the heavy chain (VH) and two variable domains of the light chain (VL) of an intact immunoglobulin protein, i.e. it comprises two Fab fragments. Additionally, F(ab')2 molecules comprise a S-S linkage in the antibody hinge region which combines the Fab fragments. The term "Fab' fragment" as used herein refers to fragments derived from "F(ab')2" molecules, preferably fragments comprising the S-S linkage in the antibody hinge region. The term "Fv fragments" as used herein refers to antibody fragments consisting of the two variable antibody domains VH and VL; further details may be derived from Skerra and Plückthun, 1988, Science, 240: 1038-1041. The term "single chain Fv fragment (scFv) " as used herein relates to antibody fragments consisting of the two VH and VL domains linked together by a flexible peptide linker; further details may be derived from Bird and Walker, 1991, Trends Biotechnol., 9: 132-137.

Also envisaged are antibody forms such as diabodies or minibodies. Details may be derived from Hudson and Kortt, 1999, J. Immunol. Methods, 231(1-2) :177-89 or Quiocho, 1993, Nature, 362(6418): 293-294.

The antibodies may be from any animal origin including birds and mammals. Preferably, the antibodies are murine (e.g., mouse and rat), donkey, monkey, rabbit, goat, guinea pig, camel, horse, chicken, or human.

The antibodies according to the present invention are typically monospecific. In certain specific embodiments, the antibody may also be bispecific, or of a greater multispecificity.

Antibodies of the present invention may also be described or specified in terms of the epitope(s) or portion(s) of a polypeptide which they recognize or specifically bind. The epitopes may preferably be present in the RNA/DNA heterohybrids as defined herein above.

In a further step of the method of the present invention, unbound antibodies are removed. The removal may be implemented with any suitable technique. In a typical and preferred embodiment, the removal is achieved by washing the antibody-bound complex as obtained in the previous step(s) of the method. The washing step may be performed once, or it may be repeated 1, 2, 3 or more times. It is preferred to perform the washing with nuclease-free water or with any other suitable solution containing appropriate ion concentration and/or having a suitable pH, as would be known to the skilled person.

In a final step of the method of the present invention, the antibody-bound RNA/DNA heterohybrids of step (vii) as mentioned above is detected. The detection may be performed according to any suitable methodology known to the skilled person. The detection of the antibody-bound RNA/DNA heterohybrids as mentioned above is preferably based on an enzymatic reaction or a non-enzymatic reaction.

The non-enzymatic reaction may, for example, be performed via the identification of a label present on the antibody as defined herein. Such a label may, for example be any suitable label known to the skilled person, e.g. a radioactive label, a fluorescent label, a chemiluminescent label or a bioluminescent label. Examples of labels that can be conjugated to an antibody according to the invention include fluorescent dyes or metals (e.g. fluorescein, rhodamine, phycoerythrin, fluorescamine), chromophoric dyes (e.g. rhodopsin), chemiluminescent compounds (e.g. luminal, imidazole), fluorescent polypeptides (e.g. green fluorescent protein (GFP)) and bioluminescent proteins (e.g. luciferin, luciferase), and contrast agents such as USPIOS or 19Fluor. Further labels which may be used within the context of the present invention include 6-FAM, HEX, TET, ROX, Cy3, Cy5, Texas Red or Rhodamine, TAMRA, Dabcyl, Black Hole Quencher, BHQ-1 or BHQ-2 . The antibody may also be labeled with radioisotopes e.g. ³H, ¹⁴C, ³²P, ³³P, ³⁵S, ¹²⁵I, ¹¹C, ¹³N, ¹⁵O, ¹⁸F, ⁶⁴Cu, ⁶²Cu, ¹²⁴I, ⁷⁶Br, ⁸²Rb, ⁶⁸Ga or ¹⁸F. Particularly preferred is a chemiluminescent detection.

More preferably, the detection is based on a chemiluminescent reaction of an acridinium ester. In general, acridinium esters can be stimulated to produce light in the presence of dilute alkaline hydrogen peroxide. Moreover, formed N-methylacridone molecules are typically resistant to quenching before radiation. The chemiluminescent label, in particular the acridinium ester, may be conjugated or covalently linked to the antibody as described herein at any suitable position. Examples of suitable acridinium dyes comprise NSP-DMAE, HQYAE, TSPAE, NSP-LEZAE, Rhodamine-2-AM-DMAE or CNF-2-AM-DMAE.

The detection of the antibody-bound RNA/DNA heterohybrids as mentioned above via an enzymatic reaction may involve the presence of an enzymatic label. Preferred enzymatic activities or labels are horseradish peroxidase (HRP), alkaline phosphatase (AP), glucose oxidase (GOD) or esterase. The enzymatic activities may accordingly be conjugated or covalently linked to the antibody as described herein at any suitable position.

The enzyme-based detection further requires a suitable substrate which is converted by an enzymatic activity as mentioned above. For example, for horseradish peroxidase (HRP) there are several different types of substrates which may be chosen in accordance with the particular assay and mode of detection. One option is the use of chromogenic HRP substrates, which remain in solution and become colored after reaction with HRP. Typically used chromogenic HRP substrates include 3,3',5,5'-tetramethylbenzidine (TMB) and 2,2'-azino-di-[3-ethylbenzthiazoline-6-sulfonic acid] (ABTS). After oxidation by HRP, some substrates precipitate. An oxidized HRP substrate may, for example, form a precipitate that is colored, electron-dense, or luminescent. An example of a suitable HRP substrate that is both, colored and electron-dense, which is envisaged by the present invention, is 3,3'-diaminobenzidine (DAB). Further envisaged is the use of the enhanced chemiluminescence (ECL) technology, e.g. as commercialized by BioRad Inc.

Suitable substrates for alkaline phosphatase (AP) include p-Nitrophenyl phosphate (PNPP) and 4-Methylumbelliferyl phosphate (4-MUP) . The use of PNPP is based on the principle that when alkaline phosphatase and PNPP are reacted, a yellow water-soluble reaction product is formed. This reaction product can be detected since it absorbs light at 405 nm. 4-MUP is a substrate for alkaline phosphatase that forms the soluble fluorescent reaction product methylumbelliferone. Fluorescence can be measured with excitation at 360 nm and emission at 440 nm. The fluorescent end product may, for example, be observed using a UV light source.

The enzymatic test with glucose oxidase (GOD} is based on the oxidation of glucose by glucose oxidase, i.e. the oxygen-dependent oxidation of the C1 carbon atom of glucose, resulting in the provision of gluconolactone and hydrogen peroxide. Hydrogen peroxide may subsequently be reduced in a downstream color reaction performed by a peroxidase (POD) such as HRP. Alternatively, the produced hydrogen peroxide may be measured electrochemically and thus be quantified.

Suitable substrates for esterase based tests include 5-Bromo-4-chloro-3-indolyl acetate, 4-Methylumbelliferyl acetate, 1-Naphthyl acetate, 1-Naphthyl butyrate, 4-Nitrophenyl dedecanoate, 4-Nitrophenyl myristate, 4-Nitrophenyl butyrate p-aminophenylbutyrate, 5-(and 6-)carboxy-2',7'-dichlorofluorescein diacetate or 4-Nitrophenyl octanoate.

The detection may be performed according to any suitable assay format, in particular an immunobiological assay. Examples of envisaged immunobiological assays in which the target RNA polynucleotide according to the present invention may be used include competitive and non-competitive assay systems using techniques such as western blots, radioimmunoassay like RIA (radio-linked immunoassay), ELISA (enzyme linked immunosorbent assay), "sandwich" immunoassays, immunoradiometric assays, fluorescent immunoassays, e.g. FIA (fluorescence-linked immunoassay), chemiluminescence immunoassays and electrochemiluminescence immunoassay (ECLIA) or suitable derivatives thereof. Details and further features of such assays would be known to the skilled person or can be derived from suitable literature sources such as the ebook Assay Guidance Manual, edited by G. Sitta Sittampalam, Bethesda (MD) : Eli Lilly & Company and the National Center for Advancing Translational Sciences; 2004 at http://www.ncbi.nlm.nih.gov/books/NBK53196/, in particular section Immunoassay Methods, Karen L. Cox et al., Eli Lilly & Company, Indianapolis, IN, of 2012, or later updated versions thereof. In specific embodiments, the assay format is an ELISA. The term "ELISA" or "ELISA assay" as used herein relates to a solid-phase enzyme immunoassay (EIA) which is generally used to detect the presence of a compound in a sample, which is typically liquid, or has been brought into solution, using one or more antibodies. The ELISA can be used as a diagnostic tool, e.g. in medicine, or as quality-control check.

Typically, antigens present in the RNA/DNA heterohybrids as defined above are attached to a solid phase such as a surface via magnetic beads or to said magnetic beads which may be attracted to a compatible surface as described herein. Subsequently, a specific antibody is applied over the surface or the beads or the surface so it can bind to the antigen. This antibody may be linked to an enzyme, e.g. as defined herein above, and, in the final step, a substance containing the enzyme's substrate is added. The subsequent reaction produces a detectable signal, for example a color change in the substrate.

In particularly preferred embodiments the assay is performed in accordance with and/or on immunoassay platforms such as Centaur or Atellica as commercialized by Siemens Healthineers. Further information on these platforms may be derived, for example, from https://www.healthcare.siemens.de/immunoassay/systems/advia-c entaur-xpt (visited on October 24, 2018) or https://www.healthcare.siemens.com/integrated-chemistry/syste ms/atellica-solution-analyzers (visited on October 24, 2018), or from the document "ADVIA Centaur XPT Immunoassay System Technical Specifications" which can be derived from suitable internet repositories such as https://static.healthcare.siemens.com/siemens_hwem-hwem_ssxa_ websites-context-root/wcm/idc/groups/public/@global/@lab/docu ments/download/mda2/otmw/~edisp/170168-gc1_updates_advia_cent aur_xpt_ss_fnl2-03931885.pdf (visited on October 25, 2018) or from the document "Atellica IM 1300 Analyzer and Atellica IM 1600 Analyzer Technical Specifications" which can be derived from suitable internet repositories such as https://static.healthcare.siemens.com/siemens_hwem-hwem_ssxa_ websites-context-root/wcm/idc/groups/public/@global/@lab/@cor elab/documents/download/mda4/mtgy/~edisp/30-17-10702-01-76_at ellica_im_1300-1600_ss_final_v2-05269699.pdf (visited on October 25, 2018).

The detection according to the present invention may be a qualitative detection, a semi-quantitative detection or a quantitative detection.

The term "qualitative detection" as used herein means that the method of the present invention is capable of indicating whether a specific target RNA polynucleotide is present or not.

Accordingly, by detecting a signal via the bound antibody, it can de deduced that the target RNA polynucleotide is present, e.g. in a mixture of different RNA polynucleotides as described above.

In the preferably envisaged ELISA a qualitative assay means that the assay can be used to achieve a yes or no answer indicating whether a target RNA polynucleotide (via the RNA/DNA heterohybrids as defined herein above) is present in a sample. This may be achieved via a comparison versus a sample containing no target RNA polynucleotide or an unrelated control target RNA polynucleotide.

The term "semi-quantitative detection" as used herein means that the method of the present invention is capable of indicating whether a specific target RNA polynucleotide is present above a certain threshold with respect to its numbers or amount or concentration in a solution. The threshold may be suitably defined as would be known to the skilled person. For example, by detecting a signal in an assay on the basis of the antibody as described herein, it can de deduced that the target RNA polynucleotide which is present, e.g. in a mixture of different RNA polynucleotides as described above, has surpassed a certain predefined limit with respect to its numbers, amount or concentration. The format also allows to compare relative levels of target RNA polynucleotides between samples, in particular between samples used within the same assay format, e.g. ELISA, or assays performed on the platforms Centaur or Atellica as mentioned herein above.

The term "quantitative detection" as used herein means that the method of the present invention is capable of indicating the approximate or exact numbers, amount or concentration of a specific target RNA polynucleotide, or of portions within said target RNA polynucleotide, used for the method. For the quantitative detection suitable control and/or calibration steps are required. Typically, the quantitative detection involves the interpretation of signals in comparison to a standard curve (e.g. a serial dilution of a known, purified target RNA polynucleotide) in order to precisely calculate the concentrations of target RNA polynucleotide in various samples. For example, in an ELISA format, the signal data obtained may be graphed with optical density vs. log concentration to produce a sigmoidal curve. Known concentrations of target RNA polynucleotide may be used to produce a standard curve. This data may subsequently be used to measure the concentration of unknown samples by comparison to the linear portion of the standard curve. This can advantageously be done directly on the graph or with suitable curve fitting software known to the skilled person. It is preferred that for quantitative measurements DNA catchers in the amount of up to 100000 are used. Based on positive calibrators a concertation curve of target RNA molecules to detectable antibody signals, e.g. from acridinium esters, may be defined, which would allow for a suitable measurement of data. It is particularly preferred to use the quantitative measurement for the detection of viral load, e.g. of HIV or HCV viral load.

It is further preferred that the quantitative detection is performed by averaging the triplicate of the standards readings and by deducting the reading of the blank control sample. Subsequently, a standard curve is plotted and the line of best fit is identified so that the concentration of the samples can be determined. Any dilutions made need to be adjusted for at this stage. Alternatively, the signal data may be plotted using semi-log, log/log, log/logit or derivatives thereof in 4 or 5 parameter logistic models. Using software based/automated solutions suitable graphing approaches may be implemented. The approach further envisages the use of linear regression, e.g. within a software package, which allows for additional control possibilities. Further details would be known to the skilled person or can be derived from suitable literature sources such as the document Laboratory Procedure Manual: Complex PSA using Advia Centaur in Serum NHANES 2007-2008 of the University of Washington Medical Centre.

In very specific embodiments detected target RNA polynucleotides, i.e. RNA polynucleotides comprising a target specific sequence, may subsequently be extracted, optionally be purified, stored and/or used for additional activities.

The present invention further envisages, in specific embodiments, the optional use or implementation of one or more additional washing or removal steps between or after any one or more of the above mentioned steps as mentioned above. These removal steps may be implemented with any suitable technique, e.g. by removing or relocating the reactants, inhibiting or destroying molecules. In a typical and preferred embodiment, the removal is achieved by washing as described herein above. The washing step may be performed once, or it may be repeated 1, 2, 3 or more times. It is preferred to perform the washing with nuclease-free water or with any other suitable solution containing appropriate ion concentration and/or having a suitable pH, as would be known to the skilled person.

In preferred embodiments, one or more of the removal or washing steps as mentioned herein above, including the removal steps for the unbound mature crRNAs, unbound catalytically inactive crRNA-guided RNA-binding protein or the unbound antibodies, may be carried out with the help of a magnet capable of collecting magnetic beads, e.g. connected to the target RNA polynucleotide as mentioned above. The beads may subsequently be washed with a continuous water flow, followed by the creation of vacuum which removes the water. It is particularly preferred that the washing steps, including any washing step as mentioned herein above, are performed in accordance with procedures described for the Centaur or Atellica assay platforms of Siemens Healthineers as described herein above or known to the skilled person.

In a further aspect the present invention relates to a kit comprising one or more DNA catcher polynucleotides which are complementary to at least a portion of the target RNA polynucleotide, a mature crRNA (CRISPR RNA) molecule which is specific for a target sequence on the target RNA polynucleotide, a catalytically-inactive crRNA-guided RNA-binding protein which is guided by said mature crRNA, and an antibody binding to RNA/DNA heterohybrids between said target RNA polynucleotide and said DNA catcher polynucleotides. The kit is preferably used for detection of a target RNA polynucleotide, e.g. in a mixture of RNA polynucleotides. The features of the methods as defined herein above apply also to the kit of the present invention. The kit may, for example, comprise reagents and components as defined in one or more steps of the present methods. The kit may, in general, comprise suitable buffer solutions, labels or washing liquids etc. The kit may also comprise suitable substrates for the detection of the bound antibody, e.g. enzymatic substrates as defined herein above, or substrates for non-enzymatic detection as described herein above. Furthermore, the kit may comprise an amount of a known nucleic acid molecule, e.g. RNA polynucleotide or protein, which can be used for a calibration of the kit or as an internal control. Corresponding ingredients would be known to the skilled person.

Additionally, the kit may comprise an instruction leaflet and/or may provide information as to its usage etc.

Also envisaged is an apparatus performing the abovementioned method steps. The apparatus may, for example, be composed of different modules which can perform one or more steps of the method of the present invention. These modules may be combined in any suitable fashion, e.g. they may be present in a single place or be separated. Also envisaged is the performance of the method at different points in time and/or in different location. Some steps of the method as defined herein may be followed by breaks or pauses, wherein the reagents or products etc. are suitably stored, e.g. in a freezer or a cooling device. In case these steps are performed in specific modules of an apparatus as defined herein, said modules may be used as storage vehicle. The modules may further be used to transport reaction products or reagents to a different location, e.g. a different laboratory etc.

In a further aspect the present invention relates to the use of one or more DNA catcher polynucleotides which are complementary to at least a portion of a target RNA polynucleotide, a mature crRNA (CRISPR RNA) molecule which is specific for a target sequence on the target RNA polynucleotide, a catalytically-inactive crRNA-guided RNA-binding protein which is guided by said mature crRNA and an antibody binding to RNA/DNA heterohybrids between said target RNA polynucleotide and said DNA catcher polynucleotide(s) for the detection of a target RNA polynucleotide. The use is preferably for an RNA polynucleotide detection assay. The assay may comprise, for example, the step of binding a DNA catcher polynucleotide which is complementary to at least a portion of a target RNA polynucleotide to said target RNA polynucleotide, the step of binding a mature crRNA (CRISPR RNA) molecule which is specific for a target sequence to the target RNA polynucleotide, and the step of binding a catalytically-inactive crRNA-guided RNA-binding protein to the target sequence via the mature crRNA. In further embodiments, the present invention also envisages the use of an antibody as defined herein above, for the detection of bound RNA/DNA heterohybrids as described above. The assay may accordingly also comprise the antibody binding and subsequent detection step as outlined above .

The features of the methods as defined herein above apply also to the use or assay as mentioned above.

Turning now to FIGURE 1, a schematic illustration of the steps for an assay for detecting a target RNA polynucleotide according to an embodiment of the present invention is provided. In a first step a target RNA polynucleotide 1 is provided. Subsequently (4), a mature crRNA (CRISPR RNA) molecule 2 and a catalytically-inactive crRNA-guided RNA-binding protein 3, wherein said crRNA-guided RNA-binding protein is biotinylated, are bound to the target RNA polynucleotide 1. In a next step (7) a magnetic bead 6, which comprises one or more streptavidin molecules, is bound to the protein-RNA polynucleotide-complex 8. In a subsequent step (15), a pool 9 of different targeted DNA catcher molecules 10, 11, 12, 13, 14 is added to the reaction mixture. The different targeted DNA catcher molecules 10, 11, 12, 13 bind to complementary stretches of the target RNA polynucleotide 1. In a further step (17) an antibody, which is specific for RNA/DNA heterohybrids 16 is bound to the complex of the previous step (vi) . The antibody comprises a label. Finally, the bound antibodies 18 can be detected.

The figure is provided for illustrative purposes. It is thus understood that the figure is not to be construed as limiting. The skilled person in the art will clearly be able to envisage further modifications of the principles laid out herein.

### LIST OF REFERENCE NUMERALS

- 1: Target RNA polynucleotide
- 2: Mature crRNA (CRISPR RNA)
- 3: Catalytically-inactive crRNA-guided RNA-binding protein
- 4: Annealing of mature crRNA (CRISPR RNA) molecule to target RNA polynucleotide and binding a catalytically-inactive crRNA-guided RNA-binding protein to the complex
- 5: Complex of Target RNA polynucleotide, mature crRNA (CRISPR RNA) molecule and catalytically-inactive crRNA-guided RNA-binding protein
- 6: Magnetic bead which comprises streptavidin molecules
- 7: Binding of magnetic bead
- 8: Complex of target RNA polynucleotide, mature crRNA (CRISPR RNA) molecule, catalytically-inactive crRNA-guided RNA-binding protein and magnetic bead
- 9: Pool of targeted DNA catcher polynucleotides
- 10: Targeted DNA catcher polynucleotide
- 11: Targeted DNA catcher polynucleotide
- 12: Targeted DNA catcher polynucleotide
- 13: Targeted DNA catcher polynucleotide
- 14: Targeted DNA catcher polynucleotide
- 15: Annealing of pool of targeted DNA catcher polynucleotides to target RNA polynucleotide within the complex
- 16: Antibody which is specific to stretches of RNA/DNA heterohybrids and which is labeled
- 17: Binding of the antibody which is specific to stretches of RNA/DNA heterohybrids to the RNA/DNA heterohybrids of the complex
- 18: Detecting of antibody-bound RNA/DNA heterohybrids

## Claims

1. A method for detecting a target RNA polynucleotide, comprising the following steps:
(i) providing an RNA polynucleotide;
(ii) annealing a mature crRNA (CRISPR RNA) molecule to a specific target sequence on the target RNA polynucleotide;
(iii) binding a catalytically-inactive crRNA-guided RNA-binding protein to the complex of step (ii) to obtain a protein-RNA polynucleotide complex, wherein said crRNA-guided RNA-binding protein is biotinylated;
(iv) binding a magnetic bead, which comprises one or more streptavidin molecules, to the protein-RNA polynucleotide-complex of step (iii);
(v) removing unbound mature crRNA molecule and unbound catalytically inactive crRNA-guided RNA-binding protein;
(vi) annealing a pool of targeted DNA catcher polynucleotides to the complex of step (iv), thereby generating stretches of RNA/DNA heterohybrids between the targeted DNA catcher polynucleotides and the target RNA polynucleotide;
(vii) specific binding of an antibody to said stretches of RNA/DNA heterohybrids of the complex of step (vi) ;
(viii) removing unbound antibody; and
(ix) detecting the antibody-bound RNA/DNA heterohybrids of step (vii).

2. The method of claim 1, wherein said pool of targeted DNA catcher polynucleotides comprises a multitude of DNA fragments which are capable of binding to different portions of the target RNA polynucleotide.

3. The method of claim 1 or 2, wherein the said pool of targeted DNA catcher polynucleotides comprises different groups of targeted DNA catcher polynucleotides, wherein each group comprises identical targeted DNA catcher polynucleotides and wherein said targeted DNA catcher polynucleotides of said different groups bind to different portions of the target RNA polynucleotide.

4. The method of claim 3, wherein said different groups of targeted DNA catcher polynucleotide cover 20% to 100% the entire length of the target RNA polynucleotide.

5. The method of anyone of claims 1 to 4, wherein said targeted DNA catcher polynucleotide is at least partially complementary to a specific target sequence on the target RNA polynucleotide.

6. The method of claim 5, wherein the complementary section between the targeted DNA catcher polynucleotide and the target RNA polynucleotide comprises between 12 to 30 nucleotides, preferably 20 nucleotides.

7. The method of any one of claims 1 to 6, wherein said antibody is specific for RNA/DNA heterohybrids, wherein said antibody is preferably a monoclonal antibody.

8. The method of any one of claims 1 to 7, wherein the catalytically-inactive crRNA-guided RNA-binding protein is a Cas protein.

9. The method of claim 8, wherein the Cas protein is a member of the family of Cas12 or Cas13 proteins, preferably a Cas13 protein or a derivative thereof.

10. The method of any one of claims 1 to 9, wherein said detection of the antibody-bound RNA/DNA heterohybrids of step (vii) is based on an enzymatic reaction or a non-enzymatic reaction.

11. The method of claim 10, wherein said enzymatic reaction is an enzymatic reaction of horseradish peroxidase (HRP), alkaline phosphatase (AP), esterase or glucose oxidase (GOD), wherein said HRP, AP, esterase or GOD is covalently linked to the antibody.

12. The method of claim 10, wherein said non-enzymatic reaction is a chemiluminescent reaction of an acridinium ester, wherein said acridinium ester is covalently linked to the antibody.

13. The method of any one of claims 1 to 12, wherein said detection is a quantitative detection or a semi-quantitative detection.

14. A kit for detecting a target RNA polynucleotide comprising one or more DNA catcher polynucleotides which are complementary to at least a portion of the target RNA polynucleotide, a mature crRNA (CRISPR RNA) molecule which is specific for a target sequence on the target RNA polynucleotide, a catalytically-inactive crRNA-guided RNA-binding protein which is guided by said mature crRNA, and an antibody binding to RNA/DNA heterohybrids between said target RNA polynucleotide and said DNA catcher polynucleotides.

15. Use of one or more DNA catcher polynucleotides which are complementary to at least a portion of a target RNA polynucleotide, a mature crRNA (CRISPR RNA) molecule which is specific for a target sequence on the target RNA polynucleotide, a catalytically-inactive crRNA-guided RNA-binding protein which is guided by said mature crRNA and an antibody binding to RNA/DNA heterohybrids between said target RNA polynucleotide and said DNA catcher polynucleotide(s) for the detection of a target RNA polynucleotide.
